(19) Europäisches Patentamt
European Patent Office
Office européen des brevets

(11) **EP 3 023 504 B1**

(12) **EUROPEAN PATENT SPECIFICATION**

(45) Date of publication and mention
of the grant of the patent:
**02.10.2019 Bulletin 2019/40**

(51) Int Cl.:
***C12Q 1/68*** *(2018.01)* ***G16B 40/00*** *(2019.01)*

(21) Application number: **13889435.7**

(22) Date of filing: **17.07.2013**

(86) International application number:
**PCT/CN2013/079495**

(87) International publication number:
**WO 2015/006932 (22.01.2015 Gazette 2015/03)**

(54) **METHOD AND DEVICE FOR DETECTING CHROMOSOMAL ANEUPLOIDY**

VERFAHREN UND VORRICHTUNG ZUM NACHWEIS VON CHROMOSOMALER ANEUPLOIDIE

PROCÉDÉ ET DISPOSITIF DE DÉTECTION D'UNE ANEUPLOÏDIE CHROMOSOMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.05.2016 Bulletin 2016/21**

(73) Proprietor: **BGI Genomics Co., Limited**
**Guangdong 518083 (CN)**

(72) Inventors:
- **ZHENG, Jing**
  **Shenzhen**
  **Guangdong 518083 (CN)**
- **ZHANG, Chunlei**
  **Shenzhen**
  **Guangdong 518083 (CN)**
- **CHEN, Shengpei**
  **Shenzhen**
  **Guangdong 518083 (CN)**
- **JIANG, Haojun**
  **Shenzhen**
  **Guangdong 518083 (CN)**
- **XIE, Weiwei**
  **Shenzhen**
  **Guangdong 518083 (CN)**
- **CHEN, Fang**
  **Shenzhen**
  **Guangdong 518083 (CN)**

(74) Representative: **Hoffmann Eitle**
**Patent- und Rechtsanwälte PartmbB**
**Arabellastraße 30**
**81925 München (DE)**

(56) References cited:
WO-A2-2005/039389    WO-A2-2010/033578
CN-A- 1 376 282       CN-A- 101 849 236
CN-A- 102 753 703     CN-A- 103 003 447

- Amy Swanson ET AL: "Molekulargenetische und zytogenetische Diagnostik/ Redaktion: H.-G. Klein Molecular-Genetic and Cytogenetic Diagnostics", J Lab Med, 1 January 2012 (2012-01-01), pages 269-275, XP055348579, DOI: 10.1515/labmed-2012-0029 Retrieved from the Internet: URL:https://www.degruyter.com/downloadpdf/ j/labm.2012.36.issue-5/labmed-2012-0029/la bmed-2012-0029.pdf
- DESHENG LIANG ET AL: "Non-invasive prenatal testing of fetal whole chromosome aneuploidy by massively parallel sequencing", PRENATAL DIAGNOSIS, vol. 33, no. 5, 9 May 2013 (2013-05-09), pages 409-415, XP055147281, ISSN: 0197-3851, DOI: 10.1002/pd.4033
- H. CHRISTINA FAN ET AL: "Sensitivity of Noninvasive Prenatal Detection of Fetal Aneuploidy from Maternal Plasma Using Shotgun Sequencing Is Limited Only by Counting Statistics", PLOS ONE, vol. 5, no. 5, 1 January 2010 (2010-01-01) , page e10439, XP055026436, ISSN: 1932-6203, DOI: 10.1371/journal.pone.0010439
- J E WEBER ET AL: "Test statistics for detecting aneuploidy and hyperdiploidy.", ANAL QUANT CYTOL HISTOL., vol. 7, no. 2, 1 June 1985 (1985-06-01), pages 131-139, XP055346916,

**(Cont. next page)**

EP 3 023 504 B1

• CHIU, R.W. ET AL.: 'Noninvasive Prenatal Diagnosis of Fetal Chromosomal Aneuploidy by Massively Parallel Genomic Sequencing of DNA in Maternal Plasma' PNAS vol. 105, no. 51, 23 December 2008, pages 20458 - 20463, XP055284693

**Description**

**BACKGROUND**

**Technical Field**

The present invention relates to the technical fields of genomics and bioinformatics, and particularly to a method and a device for detecting chromosomal aneuploidy.

**Related Art**

A chromosome is a primary substance constituting a nucleus. The somatic chromosome of a normal person has a number of 46, and has a certain morphology and structure. A karyotype generally refers to a characteristic of the chromosomal phenotype, e.g., quantity, length and the like. The karyotype detection is capable of reflecting chromosomal abnormity to a greater degree. For example, aneuploidy (deletion or duplication) of a certain chromosome has an important role in genetic studies, e.g., the detection of the fetal chromosome karyotype facilitates the reduction of birth risk.

Prenatal detection techniques commonly used at present are divided into non-invasive prenatal detection techniques and invasive prenatal detection techniques. Wherein, the non-invasive prenatal detection techniques include: 1) detection of pregnancy serum and urine ingredients by utilizing serum labels such as alpha fetoprotein (AFP), free $\beta$-human chorionic gonadotrophin ($\beta$-HCG) and pregnancy-associated plasma protein-A (PAPP-A), so as to calculate the risk of Downs syndrome; 2) visual screening of fetuses by means of a physical method, e.g., B ultrasound, X-ray, CT, magnetic resonance and the like; and 3) preimplantation genetic diagnosis (PGD) for carrying out genetic analysis on gametes or embryos before transferred into a uterine cavity, and the like. The invasive prenatal detection techniques include villus biopsy at the early pregnant stage, fetal cordocentesis at the middle pregnant stage, amniocentesis, embryoscopy and embryo biopsy, and the like.

At present, detection results from the non-invasive prenatal detection techniques are not adequately reliable, with both too high false positive rates and false negative rates. Though the invasive prenatal detection technique has high accuracy rating, risks are easily brought to pregnant women and fetuses, e.g., abortion or amniotic cavity inflammation is caused.

WO 2005/039389 discloses methods of sequence-based karyotyping.

**SUMMARY**

According to one aspect of the present invention, there is provided a method for detecting chromosomal aneuploidy, including steps as follows: acquiring the distribution of the sequencing results of the test samples on a reference sequence, wherein the test samples comprise target samples derived from M target individuals and control samples derived from N normal individuals, M and N are positive integers, the sequencing results include a plurality of sequence reads, a plurality of windows are divided on the reference sequence, and the distribution of the sequencing results of the test sample on the reference sequence manifests as the number of sequence reads $r(i,j)$ falling within each of the windows, wherein i is the serial number of the windows, j is the serial number of the test samples, and i and j are positive integers; calculating the relative sequence number $R(i,j) = r(i,j) / rp(j)$ of each test sample in each of the windows, wherein $rp(j)$ is an average value of $r(i,j)$ of sample j; calculating the deviation statistic $Z(i,j) = [R(i,j) - mean(i)] / sd(i)$ of each target sample in each window, wherein $mean(i)$ is an average value of $R(i,j)$ in window i, and $sd(i)$ is a standard deviation of $R(i,j)$ in window i; and comparing the average value $Zp(cj)$ of $Z(i,j)$ on chromosome c of the target samples with a deviation threshold of the chromosome c, as defined in claim 1, and determining whether there is a deletion or duplication in the chromosome c according to the comparison results, wherein the deviation threshold is set according to the deviation statistic of all the normal individuals on the chromosome c.

According to another aspect of the present invention, a device for detecting chromosomal aneuploidy is provided, which includes a data input unit, configured to input data; a data output unit, configured to output data; a storage unit, configured to store data, and containing an executable program therein; a processor, in data connection with the data input unit, the data output unit and the storage unit, and configured to execute the executable program stored in the storage unit, wherein the execution of the program includes performing the method for detecting chromosomal aneuploidy.

According to still another aspect of the present invention, there is provided a computer readable storage medium, configured to store a program executable by a computer. Those of ordinary skill in the art can understand that, when the program is executed, all or a part of steps of the above method for detecting chromosomal aneuploidy may be performed by relevant hardware under instruction. The storage medium mentioned may include a read-only memory, a random access memory, a magnetic disk or an optical disk and the like.

Difference between a test sample and a reference sequence is reflected by a design of the deviation statistic according to the method of the present invention. Then, whether the target sample has a chromosomal deletion or duplication is determined based on the deviation threshold determined according to the normal sample, to provide a means for detecting chromosomal aneuploidy based on the sequencing technique, which can sensitively detect abnormality in the number of any chromosome.

## BRIEF DESCRIPTION OF THE DRAWINGS

The above and/or additional aspects and advantages of the present invention will become evident and easy to understand from the description of the embodiments in conjunction with the following accompanying drawings, wherein:

FIG. 1 is a schematic flowchart of a detection method according to one embodiment of the present invention;

FIG. 2 is a schematic flowchart of a window dividing method according to another embodiment of the present invention; and

FIG. 3 is a schematic flowchart of a GC calibrating method according to another embodiment of the present invention.

## DETAILED DESCRIPTION

### Example 1

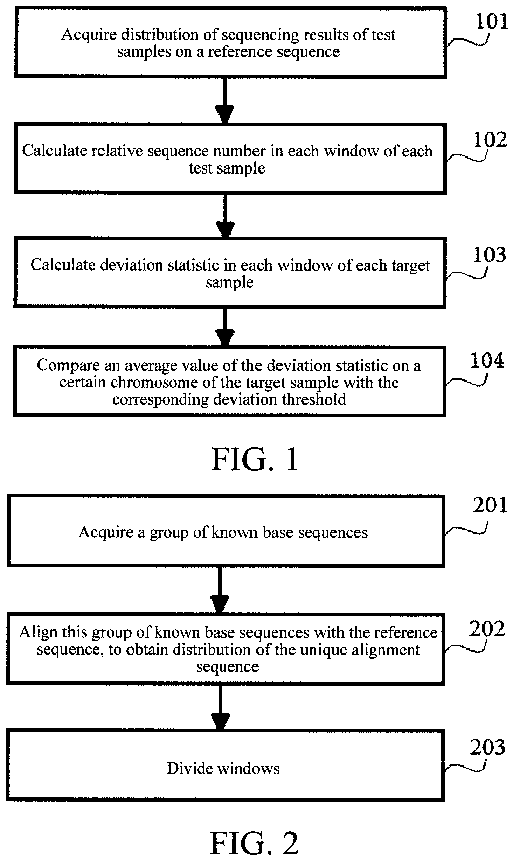

According to one embodiment of the present invention, a method for detecting chromosomal aneuploidy is provided, with reference to FIG. 1, including steps as follows:

### 101. Acquire the distribution of sequencing results of the test samples on a reference sequence.

(1) The test samples comprise target samples derived from M target individuals and control samples derived from N normal individuals, and M and N are positive integers.

The target individuals refer to individuals requiring the detection, e.g., pregnant women requiring prenatal detection, and the normal individuals refer to predetermined normal individuals. Generally, the target individual and the normal individual are the same species, preferably have approximate basic statuses. For example, if the target individual is a pregnant woman, the normal individual can be a normal pregnant woman cherishing a normal fetus at a close pregnant week.

In this embodiment, sources of the target samples and the control samples are not especially limited, and for example may be selected from the group consisting of: maternal peripheral blood, maternal urine, fetal trophoblast cells of maternal cervix, maternal cervical mucus, fetal nucleated red blood cells and the like, so long as nucleic acid samples containing genetic information of fetuses can be extracted therefrom. In this embodiment, the target sample and the control sample preferably have the same source, e.g., preferably maternal peripheral blood, as such non-invasive prenatal detection may be performed on the fetuses and the sample acquisition mode is simple and convenient. As a result of the autogeneic nucleic acids of the pregnant woman further comprised in addition to the fetal nucleic acids in the sample, in order to avoid interference thereof in the detection results, the pregnant woman herself should have no chromosomal aneuploidy problem, and of course, this judgment is generally very evident. In other embodiments, the samples may be obtained using an invasive method. For example, the samples may be derived from fetal cord blood, placenta tissues or chorionic tissues, uncultured or cultured amniotic fluid cells, villus histocytes and the like.

In this embodiment, the method and equipment for extracting, from the sample, nucleic acids for use in sequencing are not limited, and it can be performed employing various existing means, for example, commercial kits for extracting nucleic acids.

It needs to state that, if the target individuals are more than two, i.e., M≥2, each target individual may respectively form a group of test samples with N normal individuals, namely the test samples have a total number of N+1, totally M groups of test samples are obtained, and each group is subjected to detection and calculation respectively according to the method provided, or alternatively, M target individuals and N normal individuals may form a group of test samples to perform detection and calculation, i.e., the test samples have a total number of N+M. In this embodiment, an embodiment with a total number of the test samples of N+1 is preferably employed.

(2) sequencing results of the test samples include a plurality of sequence reads (i.e., reads).

Because the normal individual is selected and determined in advance, any detection or calculation data with regard to the control sample may all be generated and saved in advance. In this embodiment, this mode of presetting correlated data of the control sample is employed, the data are read and used as required, and unnecessary details are not given any longer when the data of the control sample are involved. In other embodiments, synchronous detection and calculation mode of the control sample may also be employed.

Based on the fact that embodiments of the present invention have no special dependence on the sequencing method and equipment for the samples, nucleic acids extracted from the samples are broken off, and corresponding library preparation is performed according to the sequencing method selected, followed by sequencing. For example, the third generation of sequencing platforms (Metzker ML. Sequencing technologies-the next generation. Nat Rev Genet. 2010 Jan; 11(1):31-46) may be used, including, but not limited to true single molecule sequencing techniques (True Single Molecule DNA sequencing) from Helicos Corporation, single molecule real-time sequencing (single molecule real-time (SMRTTM)) from Pacific Biosciences Corporation, and semiconductor sequencing technique from Life Technologies Corporation, and the like. In this embodiment, the semiconductor sequencing platform from the Life Technologies Corporation is preferably employed. When it is required to detect a plurality of target samples at the same time, each sample may be tagged with different barcodes, for use in the discrimination of samples during a sequencing process (Micah Hamady, Jeffrey J Walker, J Kirk Harris et al. Error-correcting barcoded primers for pyrosequencing hundreds of samples in multiplex. Nature Methods, 2008, March, Vol.5 No.3), thereby realizing sequencing of the plurality of samples at the same time. The barcodes are used in the discrimination of different samples, and have no influence on other functions of the DNA molecule with the barcode added therein. The barcode may have a length of 4 to 12 bp.

In this embodiment, the sequencing depth used in the acquisition of sequencing results of a test sample is preferably 0.2X, and a small fragment library is used with a size preferably of 100 to 300 bp. In other embodiments, the sequencing depth may be preferably 0.1X to 0.3X, simultaneously or optionally, the library has a size preferably of 50 to 500 bp. By using the above various preferred low sequencing depths and small fragment libraries, not only the data size of sequencing can be reduced to save the cost and shorten the time for detection and analysis, but also reliability and accuracy of the detection results can be ensured. For example, in one embodiment, the employment of a sequencing depth of 0.2X and a library with a size of about 100 bp can allow the sequencing result data requiring analysis to be about 5M, greatly reducing the cost for generating the data, and reducing the difficulty in the analytical calculation as well, to make it possible to complete the analysis process within 24 h, and to facilitate the shortening of result feedback time. (3) a plurality of windows are divided on the reference sequence, and the distribution of the sequencing results of the test sample on the reference sequence manifests as the number of sequence reads falling within each of the windows.

For the sake of simplicity, the number of the sequence reads in each window is denoted as r(i,j), wherein i is the serial number of the windows, j is the serial number of the test samples, and i and j are positive integers. As described above, for control samples, r(i,j) thereof may be determined and saved in advance.

The reference sequence used is a known sequence, and may be any reference template in a biologic category to which the target individual obtained in advance belongs. For example, if the target individual is a human being, a reference sequence of a human genome in the USA National Center for Biotechnology Information (NCBI) database may be selected as the reference sequence. In this embodiment, a human genome reference sequence of version 37.3 (hg19; NCBI Build 37.3) in the NCBI database is selected as the reference sequence.

The windows may be divided on the reference sequence by using various modes that allow effective statistics of the sequencing results. For example, in this embodiment, the windows are divided according to a fixed window length and a fixed window spacing, wherein the fixed window length is preferably 100 Kb, and the fixed window spacing is preferably 10 kb or 20 kb. In other embodiments, a different fixed window length and fixed window spacing may also be selected. For example, the fixed window length is preferably 1 kb to 1 Mb, and simultaneously or optionally, the fixed window spacing is preferably 1 kb to 100 kb. The window length and spacing may be set according to the abundance of fetal DNA in the sample, based on the principle that each window corresponds to one statistical magnitude and one chromosomal position, which means that the distance between the windows determines the detection precision.

When the sequencing results are aligned with the reference sequence, various alignment software, e.g., Tmap, BWA (Burrows-Wheeler Aligner), SOAP (Short Oligonucleotide Analysis Package), samtools and the like, may be used, which are not limited in this embodiment. According to the alignment software, fault tolerant (i.e., several base mismatches are permitted) or non-fault tolerant alignments may be employed. When the fault tolerant alignment is employed, generally 1 to 3 faults are permitted in 100 bp on average. When a Proton platform is employed for sequencing, generally fault tolerance alignment is employed.

## 102. Calculate relative sequence number in each window of each test sample.

For the sake of simplicity, the relative sequence number in each window of each test sample is denoted as R(i,j),

$$R(i,j) = r(i,j) / rp(j)$$

wherein, rp(j) is an average value of r(i,j) of the sample j, e.g., it can be expressed as,

$$rp(j) = [r(1,j) + \ldots + r(I,j)]/I$$

wherein, I is the number of all windows on the reference sequence.

It needs to state that, in this embodiment, subsequent analytic operation is performed using the relative sequence number after the normalization treatment, to highlight the statistical significance of the data themselves. In other embodiments, if the subsequent data analysis is performed without the employment of normalization treatment, but with the use of the treatment idea according to the present invention, and unnormalized numerical value levels are used only in the numerical analysis, calculation and comparison, such cases should be all considered as equivalent to this embodiment. In all the computational processes involved below, formulae or algorithms may also be varied employing mathematically or statistically equivalent or approximate methods, and should be equally considered as equivalent, and unnecessary details are not given any longer. This embodiment is not limited to the expression form of particular calculation formulae.

### 103. Calculate deviation statistic in each window of each target sample.

For the sake of simplicity, the deviation statistic in each window of each target sample is denoted as $Z(i,j)$,

$$Z(i,j) = [R(i,j) - mean(i)] / sd(i)$$

where, mean(i) is an average value of $R(i,j)$ in the window i, e.g., it can be expressed as,

$$mean\ (i) = [R(i,1) + \ldots + R(i,J)]/J$$

sd(i) is standard deviation of $R(i,j)$ in the window i, and one optional computing mode is:

$$sd(i) = \sqrt{\frac{1}{J-1} \sum_{j=1}^{J} [R(i,j) - mean(i)]^2}$$

Wherein, J is the number of all test samples. In this embodiment, J = 1+N. In other embodiments, if the test sample also comprises M target samples, J=M+N.

The deviation statistic $Z(i,j)$ represents that whether a deletion or duplication is occurred in the the window i of the sample j. Under the current manifestation form of the calculation formula, $Z(ij)>0$ indicates the inclination to duplication, $Z(i,j)<0$ indicates the inclination to deletion, and $Z(ij)$ of each window has relative independent statistic significance.

### 104. Compare an average value of the deviation statistic on a certain chromosome of the target sample with the corresponding deviation threshold.

(1) The deviation statistic $Z(i,j)$ is subjected to analytic alignment according to the chromosome to which it belongs, i.e., the average value $Zp(c,j)$ of $Z(i,j)$ on the chromosome c of the target sample is compared with the deviation threshold of the chromosome c,

$$Zp(c,j) = [Z(c1,j) + \ldots + Z(cI-c1+1,j)]/cI$$

Wherein, c1 is the serial number of the first window on the chromosome c of the reference sequence, and cI is the number of all windows on the chromosome c of the reference sequence.

As described above, the use other statistic values having the same or approximate meaning, e.g., an accumulated value, instead of the use of an average value, is also an equivalent practice, so long as the numerical value level of the threshold is adjusted.

(2) The chromosome c of the target sample was determined whether there is a deletion or duplication according to the comparison results. For example, if $Zp(c,j)$ exceeds an upper limit of the deviation threshold, it can be considered that the chromosome c of the target sample j has duplication (e.g., trisomy), and if $Zp(cj)$ is lower than a lower limit of the deviation threshold, it can be considered that the chromosome c of the target sample j has deletion (e.g., monosome). Therefore analytic results of digitalized karyotype of the target sample can be given, for example, "chromosomal trisomy 21," "chromosomal trisomy 18," "chromosomal trisomy 13," "deletion of X chromosome," "deletion of Y chromosome," and the like.

It needs to state that, although results of the variation detection according to embodiments of the present invention can objectively be used for determining the chromosomal aneuploidy, and thereby used in detection of genetic diseases caused thereby, e.g., fetal Downs syndrome, Edward syndrome and the like, the variation detection according to embodiments of the present invention are not necessarily used for diagnosis of diseases or associated purposes, for example, the presence of some chromosomal variation does not represent a disease risk or health condition, or can also be used in simple scientific studies of genetic polymorphism.

(3) The deviation threshold is set according to the deviation statistic on the chromosome c of all normal individuals. As described above, because the deviation threshold is obtained from the control sample, thus can be calculated and saved in advance, and when the target individual is subsequently subjected to detection, the same threshold setting can be used so long as the collection of the control sample is unchanged. Of course, if the control samples are reduced, replaced or increased, it is required to update corresponding deviation thresholds. One preferred threshold setting mode employed in this embodiment includes steps as follows.

(3.1) Control samples of N normal individuals are used as all test samples, and $Zp(c,j)$ of each control sample is calculated. A particular computational process can be performed referring to what is described in the above steps, except that the test samples comprise no target samples any longer, and thus when a deviation threshold is set, the number of all test samples is N. In order to make the deviation threshold obtained have better reliability, in this embodiment, N is preferably not less than 30.

(3.2) The corresponding $Zp(cj)$ value boundary determined to be normal is calculated according to the set test rules and confidence degrees, and used as a deviation threshold of the chromosome c. Test rules can be selected and corresponding confidence degrees can be set according to the number of control samples and the desired detection precision and the like, details of which can be performed according to the existing mode for statistical data processing. In this embodiment, U test is preferably employed, with a confidence degree of 95%, at which confidence degree, an advantage of "no false negative" is present. In other embodiments, other test rules such as T test may also be selected, and simultaneously or optionally, the confidence degree may be selected as 90% to 99.9%, e.g., 99%, 99.5%, 99.9%, and the like.

In this embodiment, a group of deviation thresholds obtained according to the above setting mode are as listed below, wherein the recorded data has a format of (serial number of the chromosome; lower limit of the threshold; upper limit of the threshold):

(1; -0.1417365; 0.1417365) (2; -0.09237466; 0.09237466)

(3; -0.1250404; 0.1250404) (4; -0.1265542; 0.1265542)

(5; -0.08148388; 0.08148388) (6; -0.119122; 0.119122)

(7; -0.1061317; 0.1061317) (8; -0.1155915; 0.1155915)

(9; -0.1004392; 0.1004392) (10; -0.1106214; 0.1106214)

(11; -0.09819914; 0.09819914) (12; -0.09005814; 0.09005814)

(13; -0.1779642; 0.1779642) (14; -0.1436377; 0.1436377)

(15; -0.1478246; 0.1478246) (16; -0.1764641; 0.1764641)

(17; -0.147383; 0.147383) (18; -0.1891044; 0.1891044)

(19; -0.3332986; 0.3332986) (20; -0.206487; 0.206487)

(21; -0.2573099; 0.2573099) (22; -0.2096556; 0.2096556)

(X-male fetus; -0.823347; 0.823347) (X-female fetus; -0.285388; 0.285388)

(Y-male fetus; -1.228768; 1.228768) (Y-female fetus; -1.217151; 1.217151)

**Example 2**

According to another embodiment of the present invention, a method for detecting chromosomal aneuploidy is provided, with basic steps being the same as in Example 1, and with difference in that, Example 1 employs a mode of dividing windows according to a fixed window length and a fixed window a spacing, whereas this embodiment divides windows employing a mode in which each window comprises the same number of the unique alignment sequence.

The unique alignment sequence refers to a sequence located in a unique position of the reference sequence. Under a circumstance where windows are divided using a mode in which "each window comprises the same number of the unique alignment sequence", when sequencing results of the test sample is aligned with the reference sequence, correspondingly, also only sequence reads with unique alignment may be counted, and sequence reads incapable of unique alignment are abandoned. This type of windows can reduce the influence of repetitive sequences, N regions and the like on detection results, to improve reliability of the detection.

This embodiment provides a method for dividing windows according to a mode in which each window comprises the same number of unique alignment sequences, with reference to FIG. 2, which includes steps as follows:
201. Acquire a group of known base sequences.

This group of base sequences can be acquired by performing whole genome sequencing on a certain know sample, e.g., one of the above control samples, or alternatively can be acquired by cutting the reference sequence according to a cut length.

When this group of known base sequences are acquired employing a mode of practical sequencing, in order to acquire a sufficient amount of the base sequences, the know sample selected may be subjected to deep sequencing, and the sequence reads obtained from the sequencing are used as this group of known base sequences. Preferably, the base sequence acquired may have length comparable to that of sequence reads obtained by sequencing of the test sample, by selecting methods of library construction and sequencing.

In the simulation of formation of this group of known base sequences employing a mode of cutting the reference sequence, the cut length may be determined first, generally according to the length of the sequence reads obtained by sequencing of the test sample. For example, the cut length may also be a fixed length value close to the length of the sequence reads of the test sample. For example, if the sequence reads of the test sample are about 250 bp, the cut length may be selected to be 200 to 300 bp. Then, the reference sequence is cut according to the cut length, e.g., HG18 or HG19 is cut according to a selected reference sequence.

202. Align this group of known base sequences with the reference sequence, to obtain the distribution of the unique alignment sequence.

203. Divide windows.

For example, K adjacent unique alignment sequences are divided into a group, thereby dividing windows covering the unique alignment sequence in each group, wherein K is a positive integer.

**Example 3**

According to another embodiment of the present invention, a method for detecting chromosomal aneuploidy is provided, with basic steps being the same as in Example 1 or 2, and with difference in that Example 1 or 2 employs the relative sequence number that is not calibrated to calculate the deviation statistic Z(ij), whereas in this embodiment, calibration on R(i,j) is performed first before the calculation of Z(i,j). For the sake of simplicity, the calibrated R(i,j) is expressed hereinafter as Ra(i,j).

In this embodiment, preferably R(i,j) is calibrated according to the GC (guanine and cytosine) content in each window of each test sample, to obtain Ra(i,j) having or approximately having normal distribution. Ra(ij) is used when Z(ij) is calculated. This is because that, as seen from an objective view, the influences of chromosomal aneuploidy (deletion or duplication) on the windows within the coverage range should be consistent, and the statistical magnitude R(ij) determined should satisfy the common statistical distribution, e.g., normal or standard normal distribution. According to the existing research results, the GC content will influence the practical sequencing result. For example, the quantity of sequence reads in a region with a high or low GC content is lower than that with a moderate GC content, which is mainly associated with the library construction method used in the sequencing process. Therefore, in order to make the detection results more reliable, R(ij) may be subjected to standardized calibration according to the GC content in each window of the test sample, to allow Ra(ij) to have a statistic rule that is for example approximately in line with normal distribution. The distribution of R(i,j) or Ra(ij) mentioned refers to the distribution of numerical values of R(i,j) described, with numerical values of R(i,j) as a horizontal coordinate, and the number of the windows containing the same numerical value of R(i,j) as a longitudinal coordinate. "The same numerical value" mentioned refers to a value within the same gear range.

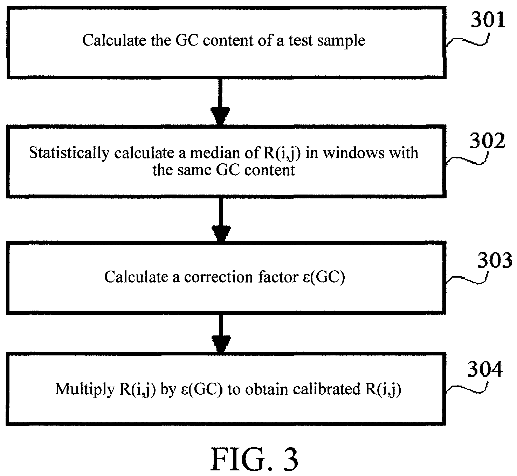

This embodiment provides a method for calibrating R(i,j) according to the GC content, with reference to FIG. 3, which includes steps as follows:

301. Calculate the GC content of the test sample.

For one test sample, the GC content in each window of the test sample can be calculated according to sequencing results. The target sample and the normal sample may be all subjected to the calibration based on the GC content, as described above, correlated data of the normal sample may be acquired and treated in advance.

302. Statistically calculate a median of R(ij) in windows with the same GC content.

The same GC content mentioned means that the GC content value lies in the same gear range. For example, in this embodiment, the gear range has a span preferably of 0.001. In other embodiments, the gear range alternatively has a span preferably from 0.0005 to 0.005.

303. Calculate a correction factor ε(GC).

Generally, a ratio of the median to a target value as the correction factor ε(GC) at a corresponding GC content. The target value is generally selected to be a value that can represent an average quantity level. For example, in this embodiment, the target value is preferably an average value of R(i,j) in all windows (including all chromosomes) of the sequencing sample.

304. Multiply R(i,j) by ε(GC) to obtain calibrated R(i,j), e.g., it can be expressed as,

$$Ra(i,j) = \varepsilon(GC) \times R(i,j)$$

Apparently, it is also possible to subject R(ij) directly to GC calibration, which is a method equivalent to the above calibration process.

Those of ordinary skill in the art can understand that, all or part of the steps of various methods in the above embodiments can be achieved by instructing related hardwares by a program that may be stored in a computer readable storage medium, which may include: a read-only memory, a random access memory, a magnetic disk or an optical disk, and the like.

According to another aspect of the present invention, a device for detecting chromosomal aneuploidy is further provided, which includes: a data input unit, configured to inputg data; a data output unit, configured to output; a storage unit, configured to store data, and containing an executable program therein; a processor, in data connection with the data input unit, the data output unit and the storage unit, and configured to execute the executable program stored in the storage unit, wherein the execution of the program includes performing all or part of the steps of various methods in the above embodiments.

Operation results according to the particular detection method of the present invention are described below in detail, in conjunction with particular target individuals. Particular parameter setting used in the following detection process is as follows:

1. the detection method of Example 3 is employed, wherein the mode in Example 1 is employed in the window setting,

2. reference sequence: a human genome reference sequence of version 37.3 (hg19; NCBIBuild37.3) in the NCBI database,

3. window length 100 Kb, window spacing 20 kb,

4. target samples: 4 cases of maternal plasma, control samples: a group of control samples determining the deviation threshold listed in Example 1.

The detection process is as follows:

1. DNA extraction and library construction: DNAs of the 4 cases of plasma samples (serial numbers of the target individuals are seen in the appended table) are extracted using a Snova DNA extraction kit (SnoMag Circulating DNA Kit). The extracted DNAs are subjected to library construction according to a proton library construction scheme after tested to be stable. Sequencing joints are added onto both ends of DNA molecules with a main band of the fragment focusing in 170 bp, and different barcode are added to each target sample when the joints are connected, for the convenience discrimination. A constructed library (with a main band at about 250 bp) is subjected to emulsion PCR into a water in oil state, to form wrapped monomolecular particles.

2. Sequencing: for the DNA samples obtained from the above 4 cases of plasma, operation is performed according to the Ion Proton instruction officially published by Life Technologies, to carry out computer sequencing, and each sample is discriminated according to the barcodes. An alignment software Tmap (available from homepage of the Life Technologies Company) is utilized, to subject sequencing results to non-fault tolerant alignment with the reference sequence, so as to obtain localization of the sequencing result on the reference sequence.

3. Data analysis: Zp(cj) of each target sample (each target sample forms a group respectively with the control samples) is calculated, and filtered using a corresponding deviation threshold, to obtain detection results exceeding the threshold.

4. Result inspection: the same 4 target individuals are analyzed according to a standard method of karyotype analysis (including processes such as amniocentesis, cell culturing, staining, and zoning), and analytic results are aligned with the results in step 3, as shown in the table as follows:

| Serial number of target individuals | Serial numbers of chromosomes | Results of standard karyotype analysis | Detection results according to the method of the present invention | Conclusion |
|---|---|---|---|---|
| CQPT01 | 21 | 47,XY,+21 | 47,XY,+21 | Consistent |
| CQPT02 | 18 | 47,XX,+18 | 47,XX,+18 | Consistent |
| CQPT03 | 13 | 47,XY,+13 | 47,XY,+13 | Consistent |
| CQPT04 | X | 45,XO | 45,XO | Consistent |

The above are only preferred examples of the present invention, and it should be understood that, these examples are only used to explain the present invention, but not limit the present invention. Those ordinarily skilled in the art can vary the above particular embodiments according to the idea of the present invention.

**Claims**

1.   A method for detecting chromosomal aneuploidy, comprising the following steps:

acquiring the distribution of the sequencing results of the test samples on a reference sequence, wherein said test samples comprise target samples derived from M target individuals and control samples derived from N normal individuals, M and N are positive integers, said sequencing results include a plurality of sequence reads, a plurality of windows are divided on said reference sequence, and said distribution manifests as the number of sequence reads $r(i,j)$ falling within each of the windows, wherein i is the number of the windows, j is the number of the test samples, and i and j are positive integers;

calculating the relative sequence number $R(i,j) = r(i,j) / rp(j)$ of each test sample in each of the windows, wherein $rp(j)$ is an average value of $r(i,j)$ of sample j;
calculating the deviation statistic $Z(i,j) = [R(i,j) - mean(i)] / sd(i)$ of each target sample in each window, wherein $mean(i)$ is an average value of $R(i,j)$ in window i, and $sd(i)$ is a standard deviation of $R(i,j)$ in window i; and

comparing the average value $Zp(c,j)$ of $Z(i,j)$ on chromosome c of the target samples with a deviation threshold of the chromosome c, and determining whether there is a deletion or duplication in the chromosome c according to the comparison results of the target samples, wherein said deviation threshold is set according to the deviation statistic of all said normal individuals on the chromosome c,
wherein said method is performed using a device for detecting chromosomal aneuploidy, the device comprising:

a data input unit, configured to input data;
a data output unit, configured to output data;
a storage unit, configured to store data, and containing an executable program therein; and
a processor, in data connection with said data input unit, the data output unit and the storage unit, and configured to execute said executable program, and wherein the execution of said program includes performing said method,

wherein said deviation threshold is set by steps as follows:

calculating Zp(c,j) of each control sample, with control samples of said N normal individuals as all test samples,

calculating the corresponding Zp(c,j) value boundary determined to be normal according to the set test rules and confidence degrees, and using this value as a deviation threshold of chromosome c.

2. The method according to claim 1, wherein sources of said target samples and the control samples are at least one selected from the group consisting of: maternal peripheral blood, maternal urine, fetal trophoblast cells of maternal cervix, maternal cervical mucus and fetal nucleated red blood cells;

the source of said target samples and the control samples is preferably maternal peripheral blood.

3. The method according to claim 1, wherein the dividing mode of said windows is selected from the group consisting of: dividing the windows according to a fixed window length and a fixed window spacing, and dividing the windows according to the mode in which each window comprises the same number of the unique alignment sequence;

said fixed window length is preferably 1 kb to 1 Mb, further preferably 100 Kb; and/or,

said fixed window spacing is preferably 1 kb to 100 kb, further preferably 5 kb to 20 kb, and more preferably 10 kb.

4. The method according to claim 3, wherein the dividing of said windows according to the mode in which each window comprises the same number of the unique alignment sequence include steps as follows:

acquiring a group of known base sequences acquired by sequencing the know samples, or by cutting the reference sequence according to a cut length determined by the length of sequence reads acquired by sequencing the test sample,

aligning said known sequence reads with the reference sequence, to acquire the distribution of the unique alignment sequence, and

dividing K adjacent only alignment sequences into a group, thereby dividing the windows covering the unique alignment sequence in each group, wherein K is a positive integer.

5. The method according to claim 1, wherein before the calculation of Z(i,j), steps as follows are further included: calibrating R(i,j) according to the GC content in each window of each test sample, to allow the calibrated R(i,j) to have or approximately have normal distribution, and using said calibrated R(i,j) when Z(i,j) is calculated.

6. The method according to claim 5, wherein said calibration of R(i,j) includes steps as follows:

for one test sample, calculating the GC content in each window of the test sample according to the sequencing results,

performing statistics for the median of R(i,j) in the window with the same GC content, wherein said same GC content means that the GC content value lies in the same gear range with a span from 0.0005 to 0.005, preferably 0.001,

taking a ratio of said median to a target value as a correction factor $\varepsilon$(GC) under a corresponding GC content, wherein said target value is preferably an average value of R(i,j) of all the windows of the test sample,

multiplying R(i,j) by $\varepsilon$(GC) to acquire calibrated R(i,j).

7. The method according to claim 1, wherein the sequencing depth used in the acquisition of sequencing results of the test sample is 0. IX to 0.3X, preferably 0.2X; and/or

a sequencing library constructed in the sequencing of the test sample has a size of 50 to 500 bp, preferably 100 to 300 bp.

8. The method according to any one of claims 1 to 7, wherein

said test rule is U test; and/or

said confidence degree is from 90% to 99.9%, and preferably 95%; and/or

said N is not less than 30.

9. The method according to any one of claims 1 to 7, wherein said sd(i) is calculated according to the following mode:

$$sd(i)=\sqrt{\frac{1}{J-1}\sum_{j=1}^{J}[R(i,j)-mean(i)]^2}\quad,$$

wherein, J is the number of all the test samples.

10. A device for detecting chromosomal aneuploidy, comprising:

> a data input unit, configured to input data;
> a data output unit, configured to output data;
> a storage unit, configured to store data, and containing an executable program therein; and
> a processor, in data connection with said data input unit, the data output unit and the storage unit, and configured to execute said executable program, wherein the execution of said program includes performing the method according to any one of claims 1 to 9.

11. A computer readable storage medium, storing a program executable by a computer, and the execution of said program comprises performing the method according to any one of claims 1 to 9.

## Patentansprüche

1. Verfahren zum Nachweis chromosomaler Aneuploidie, umfassend die folgenden Schritte:

> das Erfassen der Verteilung der Sequenzierergebnisse der Testproben auf einer Referenzsequenz, wobei die Testproben Zielproben, die von M Zielindividuen abgeleitet sind, und Kontrollproben, die von N normalen Individuen abgeleitet sind, umfassen, wobei M und N positive ganze Zahlen sind, wobei die Sequenzierergebnisse eine Mehrzahl von Sequenzlesevorgängen (sequence reads) einschließen, eine Mehrzahl von Fenstern auf die Referenzsequenz aufgeteilt werden, und sich die Verteilung als die Anzahl der Sequenzlesevorgänge $r(i,j)$ manifestiert, die in jedes der Fenster fallen, wobei i die Anzahl der Fenster ist, j die Anzahl der Testproben ist, und i und j positive ganze Zahlen sind;
> das Berechnen der relativen Sequenzanzahl $R(i,j) = r(i,j) / rp(j)$ jeder Testprobe in jedem der Fenster, wobei $rp(j)$ ein durchschnittlicher Wert von $r(i,j)$ von Probe j ist;
> das Berechnen der Abweichungsstatistik $Z(i,j) = [R(i,j) - \text{Mittelwert}(i)] / sd(i)$ jeder Zielprobe in jedem Fenster, wobei Mittelwert(i) ein durchschnittlicher Wert von $R(i,j)$ in Fenster i ist, und $sd(i)$ eine Standardabweichung von $R(i,j)$ in Fenster i ist; und
> das Vergleichen des durchschnittlichen Werts $Zp(c,j)$ von $Z(i,j)$ auf Chromosom c der Zielproben mit einem Abweichungsschwellenwert des Chromosoms c, und das Bestimmen, ob gemäß den Vergleichsergebnissen der Zielproben eine Deletion oder Duplikation in dem Chromosom c vorliegt, wobei der Abweichungsschwellenwert gemäß der Abweichungsstatistik aller normalen Individuen auf dem Chromosom c festgelegt wird, wobei das Verfahren unter Verwendung einer Vorrichtung zum Nachweis chromosomaler Aneuploidie durchgeführt wird, wobei die Vorrichtung umfasst:

> > eine Dateneingabeeinheit, die so konfiguriert ist, dass sie Daten eingibt,
> > eine Datenausgabeeinheit, die so konfiguriert ist, dass sie Daten ausgibt,
> > eine Speichereinheit, die so konfiguriert ist, dass sie Daten speichert, und die darin ein ausführbares Programm enthält; und
> > einen Prozessor, der in Datenverbindung mit der Dateneingabeeinheit, der Datenausgabeeinheit und der Speichereinheit steht, und der so konfiguriert ist, dass er das ausführbare Programm ausführt, und wobei die Ausführung des Programms das Durchführen des Verfahrens einschließt,
> > wobei der Abweichungsschwellenwert durch Schritte wie folgt festgelegt wird:

> > > das Berechnen von $Zp(c,j)$ jeder Kontrollprobe, mit Kontrollproben der N normalen Individuen als alle Testproben,
> > > das Berechnen des entsprechenden $Zp(c,j)$-Grenzwerts, der gemäß den festgelegten Testregeln und Zuverlässigkeitsgraden (confidence degrees) als normal bestimmt wird, und das Verwenden dieses Werts als einen Abweichungsschwellenwert von Chromosom c.

2. Verfahren gemäß Anspruch 1, wobei Quellen der Zielproben und der Kontrollproben mindestens eine sind, ausgewählt aus der Gruppe, bestehend aus: mütterlichem peripheren Blut, mütterlichem Urin, fetalen Trophoblastenzellen der mütterlichen Zervix, mütterlichem Zervikalschleim und fetalen kernhaltigen roten Blutkörperchen; wobei die Quelle der Zielproben und der Kontrollproben vorzugsweise mütterliches peripheres Blut ist.

**3.** Verfahren gemäß Anspruch 1, wobei die Art der Aufteilung der Fenster ausgewählt ist aus der Gruppe, bestehend aus: dem Aufteilen der Fenster gemäß einer festen Fensterlänge und einem festen Fensterabstand, und dem Aufteilen der Fenster gemäß der Art, nach der jedes Fenster die gleiche Anzahl der einzigartigen Ausrichtungssequenz (alignment sequence) umfasst;
wobei die feste Fensterlänge bevorzugt 1 kb bis 1 Mb, weiter bevorzugt 100 Kb beträgt; und/oder
wobei der feste Fensterabstand bevorzugt 1 kb bis 100 kb, weiter bevorzugt 5 kb bis 20 kb und bevorzugter 10 kb beträgt.

**4.** Verfahren gemäß Anspruch 3, wobei das Aufteilen der Fenster gemäß der Art, nach der jedes Fenster die gleiche Anzahl der einzigartigen Ausrichtungssequenz (alignment sequence) umfasst, Schritte wie folgt einschließt:

das Erfassen einer Gruppe von bekannten Basensequenzen, die durch Sequenzieren der bekannten Proben erfasst werden, oder durch Schneiden der Referenzsequenz gemäß einer Schnittlänge, die durch die Länge der Sequenzlesevorgänge (sequence reads) bestimmt wird, die durch Sequenzieren der Testprobe erfasst werden,
das Ausrichten der bekannten Sequenzlesevorgänge mit der Referenzsequenz, um die Verteilung der einzigartigen Ausrichtungssequenz zu erfassen, und
das Aufteilen von K ausschließlich angrenzenden Ausrichtungssequenzen in eine Gruppe, wodurch die die einzigartige Ausrichtungssequenz abdeckenden Fenster in jede Gruppe aufgeteilt werden, wobei K eine positive ganze Zahl ist.

**5.** Verfahren gemäß Anspruch 1, wobei vor der Berechnung von Z(i,j) Schritte wie folgt weiter eingeschlossen werden:
das Kalibrieren von R(i,j) gemäß dem GC-Gehalt in jedem Fenster jeder Testprobe, um zuzulassen, dass das kalibrierte R(i,j) eine normale Verteilung hat oder ungefähr hat, und das Verwenden des kalibrierten R(i,j), wenn Z(i,j) berechnet wird.

**6.** Verfahren gemäß Anspruch 5, wobei das Kalibrieren von R(i,j) Schritte wie folgt einschließt:

für eine Testprobe, das Berechnen des GC-Gehalts in jedem Fenster der Testprobe gemäß den Sequenzierergebnissen,
das Durchführen von Statistiken für den Median von R(i,j) im Fenster mit dem gleichen GC-Gehalt, wobei der gleiche GC-Gehalt bedeutet, dass der Wert des GC-Gehalts in dem gleichen Gangbereich (gear range) mit einer Spanne von 0,0005 bis 0,005, vorzugsweise 0,001 liegt,
das Heranziehen eines Verhältnisses des Medians zu einem Zielwert als einen Korrekturfaktor $\varepsilon(GC)$ unter einem entsprechenden GC-Gehalt, wobei der Zielwert vorzugsweise ein durchschnittlicher Wert von R(i,j) aller Fenster der Testprobe ist,
das Multiplizieren von R(i,j) mit $\varepsilon(GC)$, um kalibriertes R(i,j) zu erfassen.

**7.** Verfahren gemäß Anspruch 1, wobei die Sequenziertiefe, die beim Erfassen von Sequenzierergebnissen der Testprobe verwendet wird, 0,1X bis 0,3X, vorzugsweise 0,2X ist; und/oder
eine Sequenzierbibliothek (sequencing library), die mit dem Sequenzieren der Testprobe erstellt wird, eine Größe von 50 bis 500 bp, vorzugsweise 100 bis 300 bp aufweist.

**8.** Verfahren gemäß irgendeinem der Ansprüche 1 bis 7, wobei
die Testregel ein U-Test ist; und/oder
der Zuverlässigkeitsgrad von 90% bis 99,9% und vorzugsweise 95% ist; und/oder
das N nicht kleiner als 30 ist.

**9.** Verfahren gemäß irgendeinem der Ansprüche 1 bis 7, wobei die sd(i) gemäß der folgenden Art berechnet wird:

$$sd(i) = \sqrt{\frac{1}{J-1} \sum_{j=1}^{J} [R(i,j) - \text{Mittelwert (i)}]^2}$$,

wobei J die Anzahl aller Testproben ist.

**10.** Vorrichtung zum Nachweis von chromosomaler Aneuploidie, umfassend:

eine Dateneingabeeinheit, die so konfiguriert ist, dass sie Daten eingibt,
eine Datenausgabeeinheit, die so konfiguriert ist, dass sie Daten ausgibt,
eine Speichereinheit, die so konfiguriert ist, dass sie Daten speichert, und die darin ein ausführbares Programm enthält; und
einen Prozessor, der in Datenverbindung mit der Dateneingabeeinheit, der Datenausgabeeinheit und der Speichereinheit steht, und der so konfiguriert ist, dass er das ausführbare Programm ausführt, wobei die Ausführung des Programms das Durchführen des Verfahrens gemäß irgendeinem der Ansprüche 1 bis 9 einschließt.

**11.** Computerlesbares Speichermedium, welches ein Programm speichert, das von einem Computer ausführbar ist, und wobei die Ausführung des Programms das Durchführen des Verfahrens gemäß irgendeinem der Ansprüche 1 bis 9 umfasst.

## Revendications

**1.** Procédé pour détecter une aneuploïdie chromosomique, comprenant les étapes suivantes :

l'obtention de la distribution des résultats de séquençage des échantillons de test sur une séquence de référence, dans lequel lesdits échantillons de test comprennent des échantillons cibles dérivés de M individus cibles et des échantillons de contrôle dérivés de N individus normaux, M et N sont des nombres entiers positifs, lesdits résultats de séquençage incluent une pluralité de lectures de séquence, une pluralité de fenêtres sont divisées sur ladite séquence de référence, et ladite distribution manifeste tandis que le nombre de lectures de séquences $r(i,j)$ tombe dans chacune des fenêtres, dans lequel i est le nombre des fenêtres, j est le nombre des échantillons de test, et i et j sont des nombres entiers positifs ;
le calcul du nombre de séquence relatif $R(i,j) = r(i,j) / rp(j)$ de chaque échantillon de test dans chacune des fenêtres, dans lequel $rp(j)$ est une valeur moyenne de $r(i,j)$ de l'échantillon j ;
le calcul de la statistique de déviation $Z(i,j) = [R(i,j) - moyenne(i)] / sd(i)$ de chaque échantillon cible dans chaque fenêtre, dans lequel moyenne(i) est une valeur moyenne de $R(i,j)$ dans la fenêtre i, et sd(i) est une déviation standard de $R(i,j)$ dans la fenêtre i ; et
la comparaison de la valeur moyenne $Zp(c,j)$ de $Z(i,j)$ sur le chromosome c des échantillons cibles avec un seuil de déviation du chromosome c, et la détermination du fait qu'il y a une suppression ou une reproduction dans le chromosome c selon les résultats de comparaison des échantillons cibles, dans lequel ledit seuil de déviation est défini selon la statistique de déviation de tous lesdits individus normaux sur le chromosome c,
dans lequel ledit procédé est réalisé en utilisant un dispositif pour détecter une aneuploïdie chromosomique, le dispositif comprenant :

une unité d'entrée de données, configurée pour entrer des données ;
une unité de sortie de données, configurée pour sortir des données ;
une unité de stockage, configurée pour stocker des données, et contenant un programme exécutable dans celle-ci ; et
un processeur, en connexion de données avec ladite unité d'entrée de données, l'unité de sortie de données et l'unité de stockage, et configuré pour exécuter ledit programme exécutable, et dans lequel l'exécution dudit programme inclut la réalisation dudit procédé,
dans lequel ledit seuil de déviation est défini par étapes comme suit :

le calcul de $Zp(c,j)$ de chaque échantillon de contrôle, avec des échantillons de contrôle desdits N individus normaux comme tous les échantillons de test,
le calcul de la limite de valeur de $Zp(c,j)$ correspondante déterminée pour être normale selon les règles de test et les degrés de confiance définis, et l'utilisation de cette valeur comme un seuil de déviation du chromosome c.

**2.** Procédé selon la revendication 1, dans lequel des sources desdits échantillons cibles et des échantillons de contrôle sont au moins l'une sélectionnée à partir du groupe constitué par : du sang périphérique maternel, de l'urine maternelle, des cellules trophoblastes foetales de col de l'utérus maternel, du mucus cervical maternel et des globules rouges nucléés foetaux ;
la source desdits échantillons cibles et des échantillons de contrôle est de préférence du sang périphérique maternel.

**3.** Procédé selon la revendication 1, dans lequel le mode de division desdites fenêtres est sélectionné à partir du groupe constitué par : la division des fenêtres selon une longueur de fenêtre fixe et un espacement de fenêtre fixe, et la division des fenêtres selon le mode dans lequel chaque fenêtre comprend le même nombre de la séquence d'alignement unique ;

ladite longueur de fenêtre fixe est de préférence entre 1 kb et 1 Mb, en outre de préférence 100 Kb ; et/ou, ledit espacement de fenêtre fixe est de préférence entre 1 kb et 100 kb, en outre de préférence entre 5 kb et 20 kb, et plus de préférence 10 kb.

**4.** Procédé selon la revendication 3, dans lequel la division desdites fenêtres selon le mode dans lequel chaque fenêtre comprend le même nombre de la séquence d'alignement unique inclut des étapes comme suit :

l'obtention d'un groupe de séquences de base connues obtenues en séquençant les échantillons connus, ou en coupant la séquence de référence selon une longueur de coupe déterminée par la longueur de lectures de séquence obtenue en séquençant l'échantillon de test,

l'alignement desdites lectures de séquence connues avec la séquence de référence, pour obtenir la distribution de la séquence d'alignement unique, et

la division de K séquences d'alignement uniquement adjacentes en un groupe, divisant ainsi les fenêtres recouvrant la séquence d'alignement unique de chaque groupe, dans laquelle K est un nombre entier positif.

**5.** Procédé selon la revendication 1, dans lequel, avant le calcul de Z(i,j), des étapes comme suit sont en outre incluses : la calibration de R(i,j) selon la teneur en GC dans chaque fenêtre de chaque échantillon de test, pour permettre au R(i,j) calibré d'avoir ou d'avoir approximativement une distribution normale, et l'utilisation dudit R(i,j) calibré lorsque Z(i,j) est calculé.

**6.** Procédé selon la revendication 5, dans lequel ladite calibration de R(i,j) inclut des étapes comme suit :

pour un échantillon de test, le calcul de la teneur en GC dans chaque fenêtre de l'échantillon de test selon les résultats de séquençage,

la réalisation de statistiques pour la médiane de R(i,j) dans la fenêtre avec la même teneur en GC, dans laquelle ladite même teneur en GC signifie que la valeur de teneur en GC se trouve dans la même gamme de vitesse avec une durée allant de 0,0005 à 0,005, de préférence 0,001,

la prise d'un rapport de ladite médiane sur une valeur cible comme facteur de correction $\varepsilon(GC)$ dans une teneur en GC correspondante, dans laquelle ladite valeur cible est de préférence une valeur moyenne de R(i,j) de toutes les fenêtres de l'échantillon de test,

la multiplication de R(i,j) par $\varepsilon(GC)$ pour obtenir R(i,j) calibré.

**7.** Procédé selon la revendication 1, dans lequel la profondeur de séquençage utilisée dans l'obtention de résultats de séquençage de l'échantillon de test est de 0,1 X à 0,3 X, de préférence 0,2 X ; et/ou une bibliothèque de séquençage construite dans le séquençage de l'échantillon de test a une taille de 50 à 500 bp, de préférence de 100 à 300 bp.

**8.** Procédé selon l'une quelconque des revendications 1 à 7, dans lequel ladite règle de test est un test U ; et/ou ledit degré de confiance est de 90 % à 99,9 %, et de préférence 95 % ; et/ou ledit N n'est pas inférieur à 30.

**9.** Procédé selon l'une quelconque des revendications 1 à 7, dans lequel ledit sd(i) est calculé selon le mode suivant :

$$sd(i) = \sqrt{\frac{1}{J-1} \sum_{j=1}^{J} [R(i,j) - \text{moyenne } (i)]^2} \quad ,$$

dans lequel J est le nombre de tous les échantillons de test.

**10.** Dispositif pour détecter une aneuploïdie chromosomique, comprenant :

une unité d'entrée de données, configurée pour entrer des données ;
une unité de sortie de données, configurée pour sortir des données ;
une unité de stockage, configurée pour stocker des données, et contenant un programme exécutable dans celle-ci ; et

un processeur, en connexion de données avec ladite unité d'entrée de données, l'unité de sortie de données et l'unité de stockage, et configuré pour exécuter ledit programme exécutable, dans lequel l'exécution dudit programme inclut la réalisation du procédé selon l'une quelconque des revendications 1 à 9.

11. Support de stockage lisible par ordinateur, stockant un programme exécutable par un ordinateur, et l'exécution dudit programme comprend la réalisation du procédé selon l'une quelconque des revendications 1 à 9.

```
┌─────────────────────────────────────────────┐
│ Acquire distribution of sequencing results of test │   101
│      samples on a reference sequence          │
└─────────────────────────────────────────────┘
                      │
                      ▼
┌─────────────────────────────────────────────┐
│ Calculate relative sequence number in each window of each │   102
│                  test sample                  │
└─────────────────────────────────────────────┘
                      │
                      ▼
┌─────────────────────────────────────────────┐
│ Calculate deviation statistic in each window of each target │   103
│                   sample                      │
└─────────────────────────────────────────────┘
                      │
                      ▼
┌─────────────────────────────────────────────┐
│ Compare an average value of the deviation statistic on a │   104
│   certain chromosome of the target sample with the   │
│         corresponding deviation threshold      │
└─────────────────────────────────────────────┘
```

## FIG. 1

```
┌─────────────────────────────────────────────┐
│       Acquire a group of known base sequences  │   201
└─────────────────────────────────────────────┘
                      │
                      ▼
┌─────────────────────────────────────────────┐
│ Align this group of known base sequences with the reference │   202
│  sequence, to obtain distribution of the unique alignment   │
│                   sequence                    │
└─────────────────────────────────────────────┘
                      │
                      ▼
┌─────────────────────────────────────────────┐
│                 Divide windows                │   203
└─────────────────────────────────────────────┘
```

## FIG. 2

| Calculate the GC content of a test sample | 301 |

↓

| Statistically calculate a median of R(i,j) in windows with the same GC content | 302 |

↓

| Calculate a correction factor ε(GC) | 303 |

↓

| Multiply R(i,j) by ε(GC) to obtain calibrated R(i,j) | 304 |

## FIG. 3

**EP 3 023 504 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005039389 A

### Non-patent literature cited in the description

- **METZKER ML.** Sequencing technologies-the next generation. *Nat Rev Genet,* January 2010, vol. 11 (1), 31-46

- **MICAH HAMADY ; JEFFREY J WALKER ; J KIRK HARRIS et al.** Error-correcting barcoded primers for pyrosequencing hundreds of samples in multiplex. *Nature Methods,* March 2008, vol. 5 (3